# EUROPEAN PATENT SPECIFICATION

(11) **EP 2 341 894 B1**
(45) Date of publication and mention of the grant of the patent: **29.08.2012**
(21) Application number: 09788094.2
(22) Date of filing: 02.10.2009
(51) Int. Cl.: A61K 9/00, A61K 47/10, A61K 47/26

(54) **PHARMACEUTICAL COMPOSITION COMPRISING A PROSTAGLANDIN**
PHARMAZEUTISCHE ZUBEREITUNG EINES PROSTAGLANDIN
COMPOSITION PHARMACEUTIQUE COMPRENANT UNE PROSTAGLANDINE

(30) Priority: 07.10.2008 US 103403 P
(43) Date of publication of application: 13.07.2011
(73) Proprietor: R-Tech Ueno, Ltd., Tokyo 100-0011 (JP); Sucampo AG, 6300 Zug (CH)
(72) Inventor: HARADA, Yasuhiro, Tokyo 100-0011 (JP); KAWASAKI, Junichi, Tokyo 100-0011 (JP); HAYASHI, Tadashi, Tokyo 100-0011 (JP); UENO, Ryuji, Maryland 20854 (US)
(74) Representative: Albrecht, Thomas
(86) International application number: PCT/JP2009/067586
(87) International publication number: WO 2010/041722

(56) References cited:
- EP-A1- 1 994 933
- WO-A1-2005/023258
- US-A1- 2002 002 185
- US-A1- 2002 058 049
- US-A1- 2004 076 678

## Description

### TECHNICAL FIELD.

The present invention relates to a pharmaceutical composition comprising a prostaglandin compound that can be stored stably. The present invention also relates to a pharmaceutical composition comprising a prostaglandin compound that can be stored stably even in a polyethylene container. Further, the present invention relates to a pharmaceutical composition comprising a prostaglandin compound and a small amount of a preserving agent that can be stored stably for long term.

### BACKGROUND ART

Prostaglandins (hereinafter; referred to as PG(s)) are members of class of organic carboxylic acids, which are contained in tissues or organs of human and other mammals, and exhibit a wide range of physiological activities. PGs found in nature (primary PGs) have, as a general structural property thereof, a prostanoic acid skeleton as shown in formula (A):

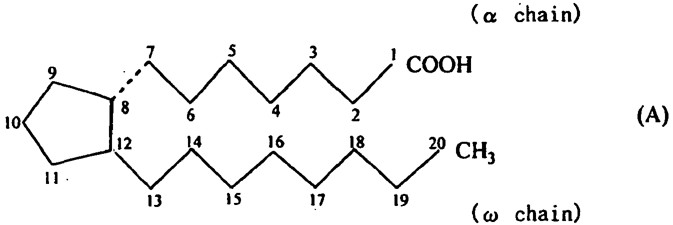

On the other hand, some synthetic PG analogues have modified skeletons. The primary PGs are classified into PGAs, PGBs, PGCs, PGDs, PGEs, PGFs, PGGs, PGHs, PGIs and PGJs on the basis of the structural property of the five membered ring moiety, and further classified into the following three types by the number and position of the unsaturated bond in the carbon chain moiety.
Type 1 (subscript 1): 13,14-unsaturated-15-OH
Type 2 (subscript 2): 5,6- and 13,14-diunsaturated-15-OH
Type 3 (subscript 3): 5,6-, 13,14-, and 17,18-triunsaturated-15-OH.

Further, PGFs are classified on the basis of the configuration of the hydroxy group at the 9-position into α type, wherein the hydroxy group is of the α-configuration and β type, wherein the hydroxy group is of the β-configuration.

Some 15-keto-PGs (PGs having an oxo group at position 15 in place of the hydroxy group) and 13,14-dihydro (PGs having a single bond between positions 13 and 14)-15-keto-PGs have been known as substances naturally produced by enzymatic actions during metabolism of the primary PGs and have some therapeutic effect. 15-keto-PGs have been disclosed in USP Nos. 5,073,569, 5,534,547, 5,225,439, 5,166,174, 5,428,062 5, 380, 709 5,886,034 6,265,440, 5,106,869, 5,221,763, 5,591,887, 5,770,759 and 5, 739, 161.

Some PG compounds have been known as drugs used in the ophthalmic field, for example, for lowering intraocular pressure or treating glaucoma. For example, 13,19-dihydro-17-phenyl-18,19,20-trinor-PGF2α isopropyl ester (general name: latanoprost), 16-(3-trifluoromethylphenoxy)-17,18,19,20-trinor-PGF2α isopropyl ester (general name: travoprost) and 17-phenyl-18,19,20-trinor-PGF2α N-ethylamide (general name: bimatoprost) have been marketed as ophthalmic solution for the treatment of glaucoma and/or ocular hypertension under the name of Xalatan®, Travatan® and Lumigan®, respectively.

Further, 15-keto-prostaglandin compound have also been known to be useful in the ophthalmic field, for example, for lowering intraocular pressure and treating glaucoma (see USPs 5,001,153, 5,151,444, 5,166,178, 5,194,429 and 5,236,907), for treating cataract (see USPs 5,212,324 and 5,686,987), for increasing the choroidal blood flow (see USP 5,221,690), for treating optic nerve disorder (see USP 5,773,471). Ophthalmic solution comprising 13,14-dihydro-15-keto-20-ethyl-PGF2α isopropyl ester (general name: isopropyl unoprostone) has been marketed under the name of Rescula® as a pharmaceutical product for the treatment of glaucoma and ocular hypertension.

Some prostaglandin compounds that have been used for the treatment of glaucoma and/or ocular hypertension are not stable when stored in polyethylene containers and therefore, have been stored in polypropylene containers. In addition, all pharmaceutical compositions used in working examples disclosed in, for example, WO00/03736 and US Publication No. 20020058049 are supplemented with a sugar alcohol, mannitol. US 2002/0002185 discloses composition comprising prostaglandin compound, non-ionic surfactant(s), non-ionic tonicity adjusting agent(s) selected form e.g. mannitol, polyethylene glycols and their mixtures.

### DISCLOSURE OF INVENTION

An object of the present invention is to provide a pharmaceutical composition comprising a prostaglandin compound that can be stored stably in a polyethylene container. Another object of the present invention is to provide a pharmaceutical composition comprising a prostaglandin compound and a relatively low amount of a preserving agent that can be stored with enough stability.

The inventor has found that a composition comprising a PG compound, a sugar alcohol and a polyol can be stored stably even in a polyethylene container and can exhibit good property of the PG compound.

The application provides the followings:
(1) A pharmaceutical composition comprising:
   (a) a prostaglandin compound as defined in claim 1, (b) a sugar alcohol as defined in claim 1, (c) a polyol as defined in claim 1, and (d) a pharmaceutically acceptable carrier wherein the composition is stored in a polyethylene container.
(2) The pharmaceutical composition of (1), wherein the prostaglandin compound is isopropyl unoprostone.
(3) The pharmaceutical composition of (1), further comprising a non-ionic surface active agent.
(4) The pharmaceutical composition of (1), which is used for the treatment of glaucoma and/or ocular hypertension or retinitis pigmentosa.
(5) The pharmaceutical composition of (1), wherein the sugar alcohol is mannitol.
(6) The pharmaceutical composition of (1), wherein polyol is glycerine.
(7) The pharmaceutical composition of (1), which is in a dosage form suitable for ocular topical administration.
(8) The pharmaceutical composition of (1), which is formulated as eye drops.
(9) The pharmaceutical composition of (1), wherein the polyethylene container is made of low density polyethylene.
(10) The pharmaceutical composition of (1), wherein the pharmaceutically acceptable carrier is water.

The nomenclature of PG compounds used herein is based on the numbering system of prostanoic acid represented in the above formula (A).

The formula (A) shows a basic skeleton of the C-20 PG compound, but the present invention is not limited to those having the same number of carbon atoms. In the formula (A), the numbering of the carbon atoms which constitute the basic skeleton of the PG compounds starts at the carboxylic acid (numbered 1), and carbon atoms in the α-chain are numbered 2 to 7 towards the five-membered ring, those in the ring are 8 to 12, and those in the ω-chain are 13 to 20. When the number of carbon atoms is decreased in the α-chain, the number is deleted in the order starting from position 2; and when the number of carbon atoms is increased in the α-chain, compounds are named as substitution compounds having respective substituents at position 2 in place of carboxy group (C-1). Similarly, when the number of carbon atoms is decreased in the ω-chain, the number is deleted in the order starting from position 20; and when the number of carbon atoms is increased in the ω-chain, the carbon atoms at the position 21 or later are named as a substituent at position 20. Stereochemistry of the compounds is the same as that of the above formula (A) unless otherwise specified.

In general, each of PGD, PGE and PGF represents a PG compound having hydroxy groups at positions 9 and/or 11, but in the present specification they also include those having substituents other than the hydroxy groups at positions 9 and/or 11. Such compounds are referred to as 9-deoxy-9-substituted-PG compounds or 11-deoxy-11-substituted-PG compounds. A PG compound having hydrogen in place of the hydroxy group is simply named as 9- or 11-deoxy compound.

As stated above, the nomenclature of PG compounds is based on the prostanoic acid skeleton. In the case the compound has similar partial structure as the primary prostaglandin compound, the abbreviation of "PG" may be used. Thus, a PG compound whose α-chain is extended by two carbon atoms, that is, having 9 carbon atoms in the α-chain is named as 2-decarboxy-2-(2-carboxyethyl)-PG compound. Similarly, a PG compound having 11 carbon atoms in the α-chain is named as 2-decarboxy-2-(4-carboxybutyl)-PG compound. Further, a PG compound whose ω-chain is extended by two carbon atoms, that is, having 10 carbon atoms in the ω-chain is named as 20-ethyl-PG compound. These compounds, however, may also be named according to the IUPAC nomenclatures.

The PG compound used in the present invention may be any substitution compound or derivative of a PG. PG compound and may include a PG1 compound having one double bond between positions 13 and 14, and a hydroxy group at position 15; a PG2 compound having one additional double bound between positions 5 and 6; and a PG3 compound having a further double bond between positions 17 and 18. In addition, a 15-keto-PG compound having oxo group at position 15 instead of the hydroxy group; a 15-deoxy PG compound having hydrogen instead of the hydroxy group at position 15; and a 15-fluoro PG compound having a fluorine at position 15 instead of the hydroxy group may also be included. Further, 13,14-dihydro compound in which the double bond between positions 13 and 14 is single bond and 13,14-didehydro-PG compound in which the double bond between the positions of 13 and 14 is triple bond may also be included. Further more, examples of the analogues including substitution compounds or derivatives of the PG compound include a PG compound whose carboxy group at the end of the α chain is esterified or amidated, or a physiologically acceptable salt thereof; a PG compound whose α or ω chain is shortened or extended than that of the primary PG; a PG compound having a side chain that having for example, 1-3 carbon atoms, on their α or ω chain; a PG compound having a substituent such as hydroxy, halogen, lower alkyl, hydroxy(lower)alkyl or oxo, or a double bond on its five membered ring; a PG compound having a substituent such as halogen, oxo, aryl and heterocyclic group on its α chain; a PG compound having a substituent such as halogen, oxo, hydroxy, lower alkoxy, lower alkanoyloxy, cyclo(lower)alkyl, cyclo(lower)alkyloxy, aryl, aryloxy, heterocyclic or heterocyclic-oxy on its ω chain; and a PG compound having shorter ω chain than that of normal prostanoic acid and having a substituent such as lower alkoxy, lower alkanoyloxy, cyclo(lower)alkyl, cyclo(lower)alkyloxy, aryl, aryloxy, heterocyclic or heterocyclic-oxy group at the end of the ω chain.

### PREFERRED EMBODIMENT TO CARRY OUT THE INVENTION

In the above formula (I), the term "unsaturated" in the definitions for R₁ and Ra is intended to include at least one or more double bonds and/or triple bonds that are isolatedly, separately or serially present between carbon atoms of the main and/or side chains. According to the usual nomenclature, an unsaturated bond between two serial positions is represented by denoting the lower number of the two positions, and an unsaturated bond between two distal positions is represented by denoting both of the positions.

The term "lower or medium aliphatic hydrocarbon" refers to a straight or branched chain hydrocarbon group having 1 to 14 carbon atoms (for a side chain, 1 to 3 carbon atoms are preferable) and preferably 1 to 10, especially 6 to 10 carbon atoms for R₁ and 1 to 10, especially 1 to 8 carbon atoms for Ra.
The term "halogen" covers fluorine, chlorine, bromine and iodine.
The term "lower" is intended to include a group having 1 to 6 carbon atoms unless otherwise specified.

The term "lower alkyl" refers to a straight or branched chain saturated hydrocarbon group containing 1 to 6 carbon atoms and includes, for example, methyl, ethyl, propyl, isopropyl, butyl, isobutyl, sec-butyl, t-butyl, pentyl and hexyl.
The term "lower alkoxy" refers to a group of lower alkyl-O-, wherein lower alkyl is as defined above.
The term "hydroxy(lower)alkyl" refers to a lower alkyl as defined above which is substituted with at least one hydroxy group such as hydroxymethyl, 1-hydroxyethyl, 2-hydroxyethyl and 1-methyl-1-hydroxyethyl.

The term "lower alkanoyloxy" refers to a group represented by the formula RCO-O-, wherein RCO- is an acyl group formed by oxidation of a lower alkyl group as defined above, such as acetyl.

The term "cyclo(lower)alkyl" refers to a cyclic group formed by cyclization of a lower alkyl group as defined above but contains three or more carbon atoms, and includes, for example, cyclopropyl, cyclobutyl, cyclopentyl and cyclohexyl.

The term "cyclo(lower)alkyloxy" refers to the group of cyclo(lower)alkyl-O-, wherein cyclo(lower)alkyl is as defined above.

The term "aryl" may include unsubstituted or substituted aromatic hydrocarbon rings (preferably monocyclic groups), for example, phenyl, tolyl, xylyl. Examples of the substituents are halogen and lower alkyl substituted by halogen, wherein halogen and lower alkyl are as defined above.
The term "aryloxy" refers to a group represented by the formula ArO-, wherein Ar is aryl as defined above.

The term "heterocyclic group" may include mono-to tri-cyclic, preferably monocyclic heterocyclic group which is 5 to 14, preferably 5 to 10 membered ring having optionally substituted carbon atom and 1 to 4, preferably 1 to 3 of 1 or 2 types of hetero atoms selected from nitrogen atom, oxygen atom and sulfur atom. Examples of the heterocyclic group include furyl, thienyl, pyrrolyl, oxazolyl, isoxazolyl, thiazolyl, isothiazolyl, imidazolyl, pyrazolyl, furazanyl, pyranyl, pyridyl, pyridazinyl, pyrimidyl, pyrazinyl, 2-pyrrolinyl, pyrrolidinyl, 2-imidazolinyl, imidazolidinyl, 2-pyrazolinyl, pyrazolidinyl, piperidino, piperazinyl, morpholino, indolyl, benzothienyl, quinolyl, isoquinolyl, purinyl, quinazolinyl, carbazolyl, acridinyl, phenanthridinyl, benzimidazolyl, benzimidazolinyl, benzothiazolyl and phenothiazinyl. Examples of the substituent in this case include halogen, and lower alkyl substituted by halogen, wherein halogen and lower alkyl group are as described above.

The term "heterocyclic-oxy group" means a group represented by the formula HcO-, wherein Hc is a heterocyclic group as described above.
The term "functional derivative" of A includes salts, preferably pharmaceutically acceptable salts, ethers, esters and amides.

Suitable "pharmaceutically acceptable salts" include salts formed with non-toxic bases conventionally used in pharmaceutical field, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt including such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

Examples of the ethers include alkyl ethers, for example, lower alkyl ethers such as methyl ether, ethyl ether, propyl ether, isopropyl ether, butyl ether, isobutyl ether, sec-butyl ether, t-butyl ether, pentyl ether and 1-cyclopropyl ethyl ether; and medium or higher alkyl ethers such as octyl ether, diethylhexyl ether, lauryl ether and cetyl ether; unsaturated ethers such as oleyl ether and linolenyl ether; lower alkenyl ethers such as vinyl ether, allyl ether; lower alkynyl ethers such as ethynyl ether and propynyl ether; hydroxy(lower)alkyl ethers such as hydroxyethyl ether and hydroxyisopropyl ether; lower alkoxy (lower)alkyl ethers such as methoxymethyl ether and 1-methoxyethyl ether; optionally substituted aryl ethers such as phenyl ether, tosyl ether, t-butylphenyl ether, salicyl ether, 3,4-di-methoxyphenyl ether and benzamidophenyl ether; and aryl(lower)alkyl ethers such as benzyl ether, trityl ether and benzhydryl ether.

Examples of the esters include aliphatic esters, for example, lower alkyl esters such as methyl ester, ethyl ester, propyl ester, isopropyl ester, butyl ester, isobutyl ester, sec-butyl ester, t-butyl ester, pentyl ester and 1-cyclopropylethyl ester; lower alkenyl esters such as vinyl ester and allyl ester; lower alkynyl esters such as ethynyl ester and propynyl ester; hydroxy(lower)alkyl ester such as hydroxyethyl ester; lower alkoxy (lower) alkyl esters such as methoxymethyl ester and 1-methoxyethyl ester; and optionally substituted aryl esters such as, for example, phenyl ester, tolyl ester, t-butylphenyl ester, salicyl ester, 3,4-di-methoxyphenyl ester and benzamidophenyl ester; and aryl(lower)alkyl ester such as benzyl ester, trityl ester and benzhydryl ester.

The amide of A means a group represented by the formula -CONR'R", wherein each of R' and R" is hydrogen, lower alkyl, aryl, alkyl- or aryl-sulfonyl, lower alkenyl and lower alkynyl, and include for example lower alkyl amides such as methylamide, ethylamide, dimethylamide and diethylamide; arylamides such as anilide and toluidide; and alkyl- or aryl-sulfonylamides such as methylsulfonylamide, ethylsulfonyl-amide and tolylsulfonylamide.

Preferred examples of L and M are hydroxy and oxo which provide a 5-membered ring structure of, so called, PGE type and PGF type.

Preferred examples of A are -COOH and its pharmaceutically acceptable salt, ester and amide.

Preferred example of B is -CH₂-CH₂- which provides a compound having a structure called as 13, 14-dihydro type prostaglandin.

Preferred example of X₁ and X₂ is hydrogen, and at least one of them is halogen, more preferably, both of them are halogen, especially, fluorine that provides a structure of, so called 16,16-difluoro type prostaglandin.

Preferred Z is =O, or 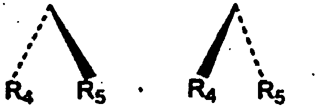 wherein one of R₄ and R₅ is hydrogen and the other is hydroxy, and more preferably, Z is =O that provides so called 15-keto type prostaglandin.

Preferred R₁ is an unsaturated or saturated, un substituted bivalent lower to medium aliphatic hydrocarbon. Preferably, R1 contains 1-10 carbon atoms and most preferably, 6-8 carbon atoms. Further, at least one carbon atom in the aliphatic hydrocarbon is optionally substituted by oxygen, nitrogen or sulfur.

Examples of R₁ include, for example, the followings:
-CH₂-CH₂-CH₂-CH₂-CH₂-CH₂-,
-CH₂-CH=CH-CH₂-CH₂-CH₂-,
-CH₂-CH₂-CH₂-CH₂-CH=CH-,
-CH₂-C=C-CH₂-CH₂-CH₂-,
-CH₂-CH₂-CH₂-CH₂-CH(CH₃)-CH₂-,
-CH₂-CH₂-CH₂-CH₂-O-CH₂-,
-CH₂-CH=CH-CH₂-O-CH₂-,
-CH₂-C=C-CH₂-O-CH₂-,
-CH₂-CH₂-CH₂-CH₂-CH₂-CH₂-CH₂-,
-CH₂-CH=CH-CH₂-CH₂-CH₂-CH₂-,
X-CH₂-CH₂-CH₂-CH₂-CH₂-CH=CH-,
-CH₂-C≡C-CH₂-CH₂-CH₂-CH₂-,
-CH₂-CH₂-CH₂-CH₂-CH₂-CH(CH₃)-CH₂-,
-CH₂-CH₂-CH₂-CH₂-CH₂-CH₂-CH₂-CH₂-,
-CH₂-CH=CH-CH₂-CH₂-CH₂-CH₂-CH₂-,
-CH₂-CH₂-CH₂-CH₂-CH₂-CH₂-CH=CH-,
-CH₂-C≡C-CH₂-CH₂-CH₂-CH₂-CH₂-, and
-CH₂-CH₂-CH₂-CH₂-CH₂-CH₂-CH(CH₃)-CH₂.

Preferred Ra is a hydrocarbon containing 1-10 carbon atoms, more preferably, 1-8 carbon atoms and most preferably, 5-7 carbon atoms. Ra may have one or two side chains each having one carbon atom.

Preferred R₂ is single bond.

Preferred R₃ is a lower alkyl, especially lower alkyl having 4-6 carbon atoms. R₃ may have one or two side chains each having one carbon atom.

The configuration of the ring and the α- and/or ω chains in the above formulae (I) and (II) may be the same as or different from that of the primary PGs. The present invention also includes a mixture of a compound having the primary type configuration and a compound of a non-primary type configuration.

The typical examples of the compounds used in the instant application include: 13,14-dihydro-15-keto-20-ethyl-PGF compound, 13,14-dihydro-15-keto-16,16-difluoro-PGE compound, 11-deoxy-13,14-dihydro-15-keto-16,16-difluoro-PGE compound and 18,19,20-trinol-17-phenyl-PGF compound, and derivatives and analogs thereof.

In the present invention, the 15-keto-PG compound may be in the keto-hemiacetal equilibrium by the formation of a hemiacetal between hydroxy at position 11 and oxo at position 15.

For example, it has been revealed that when both of X₁ and X₂ are halogen atoms, especially, fluorine atoms, the compound contains the bicyclic compound as tautometric isomer.

If such tautomeric isomers as above are present, the proportion of both tautomeric isomers varies with the structure of the rest of the molecule or the kind of the substituent present. Sometimes one isomer may predominantly be present in comparison with the other. The 15-keto-PG compound of the present invention includes both isomers.

Further, the 15-keto-PG compounds used in the invention include the bicyclic compound and analogs or derivatives thereof. The bicyclic compound is represented by the formula (III):

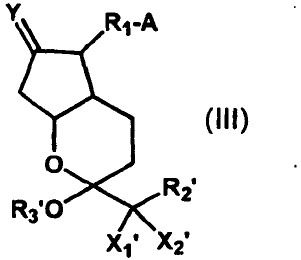

wherein, A is -CH₃, -CH₂OH, -COCH₂OH, -COOH or a functional derivative thereof;
X₁' and X₂' are hydrogen, lower alkyl, or halogen;
Y is 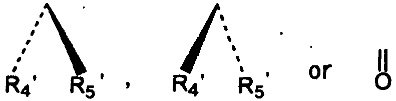 wherein R₄' and R₅' are hydrogen, hydroxy, halogen, lower alkyl, lower alkoxy or hydroxy(lower)alkyl, wherein R₄' and R₅' are not hydroxy and lower alkoxy at the same time.
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one of carbon atoms in the aliphatic hydrocarbon is optionally substituted by oxygen, nitrogen or sulfur; and
R₂' is a saturated or unsaturated lower or medium aliphatic hydrocarbon residue, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; or heterocyclic-oxy group.
R₃' is hydrogen, lower alkyl, cyclo(lower)alkyl, aryl or heterocyclic group.

Furthermore, while the compounds used in the invention may be represented by a formula or name based on keto-type compound regardless of the presence or absence of the isomers, it is to be noted that such structure or name does not intend to exclude the hemiacetal type compound.

In the present invention, any of isomers such as the individual tautomeric isomers, the mixture thereof, or optical isomers, the mixture thereof, a racemic mixture, and other steric isomers may be used in the same purpose.

Some of the compounds used in the present invention may be prepared by the method disclosed in USP Nos.5,073,569, 5,166,174, 5,221,763, 5,212,324, 5,739,161 and 6,242,485.

It has been known that 13,14-dihydro-15-keto-prostaglandin compound having the formula as shown below (Tautomer I) may be in equilibrium with its tautomeric isomer (tautomer II) (See USP 5,166,174, USP 5,225,439, USP 5,284,858, USP 5,380,709, USP 5,428,062 and USP 5, 886, 034.

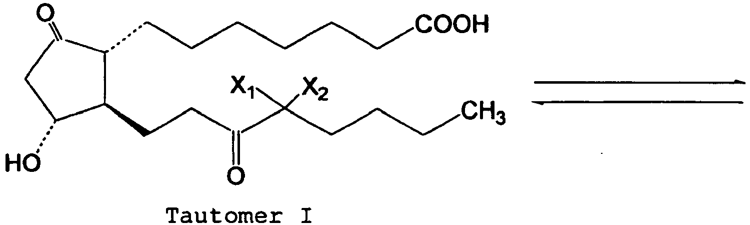

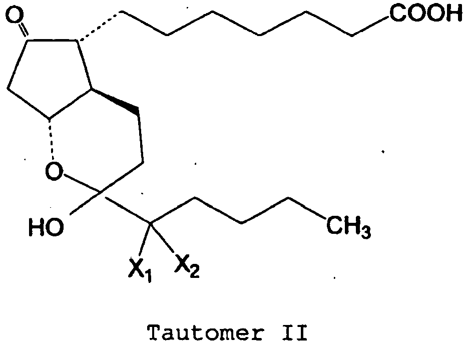

The PG compound described as above is useful for manufacturing pharmaceutical products for various uses and especially useful for the treatment of symptoms in the ophthalmic field such as glaucoma and/or ocular hypertension and retinal degeneration.

The term "treatment" or "treating" used herein refers to any means of control of a condition including prevention, cure, relief of the condition, attenuation of the condition and arrest of progression.

In the pharmaceutical composition of the present invention, the PG compound, the active ingredient, may be any of the above described compounds.
The concentration of the 15-keto-PG compound in the aqueous composition may vary depending on the specific compound being used, species, age and body weight of the subject to be treated, condition to be treated, desired therapeutic effect, administration amount and treating period and the art can determine a suitable concentration. Typically the dose of the PG compound to provide sufficient effect by systemic administration in accordance with divided dose into one to four fractions per day or under sustained condition may be 0.00001-100mg/kg per day.

According to the present invention, "pharmaceutical composition" refers to a pharmaceutical dosage form comprising the PG compound as an active ingredient and may be used as eye drop, nasal drop, ear drop, inhalant, spray, oral administerable product or injectable (intravenous, intra-arterial, subcutaneous, intramuscular, intraperitoneal and intraocular) product. The concentration of PG compound in the aqueous composition may generally be about 0.0001 to 10 w/v%, preferably, about 0.0001 to 5 w/v% and more preferably about 0.001 to 1 w/v% based on the total volume of the composition.

The sugar alcohols used in the instant application is an alcohol obtained by hydrogen reduction of the aldehyde group of a saccharide. Examples may comprise sorbitol, mannitol, maltitol, lactitol, palatinit, xylitol and erythritol; and sugar alcohol solution derived from corn starch, i.e. a mixture of sorbitol, sorbitan, mannitol and hydrogenated starch hydrolysate, hydrogenated maltose starch syrup, i.e. a mixture of maltitol, sorbitol and oligosaccharide alcohol. Mannitol is most preferable. The amount of the sugar alcohol added to the pharmaceutical composition of the instant application may generally be about 0.1-5 w/v%.

Polyols used in the present invention are polyvalent alcohols and preferably, those having two or three hydroxy groups. Preferred examples of the polyols may include glycerine, polyethyleneglycol and propyleneglycol. Glycerine is most preferable. The amount of the polyol added to the pharmaceutical composition of the instant application may generally be about 0.1-5 w/v%.

In order to satisfy the object of the present invention, i.e. to provide a stable pharmaceutical composition in which the PG compound contained in the composition is stable even if it is stored in a polyethylene container, the composition must comprise a sugar alcohol and a polyol in addition to the PG compound. The ratio of the sugar alcohol to the polyol may be in the range of about 1:10-10:1 and preferably, about 1:5-5:1.

According to the instant application, pharmaceutically acceptable carrier may be any media that can dissolve or disperse the PG compound therein. Non limited examples of the carriers may include distilled water, saline, edible oil, mineral oil and liquid paraffin, and a mixture thereof.

When the composition is provided as a ophthalmic formulation, the pharmaceutically acceptable carrier may preferably be distilled water or saline.

The pharmaceutical composition may further be added with a nonionic surface active agent to improve the solubility of the PG compound. Non-ionic surface active agent represents a surface active agent that has no group that is easily ionized. Examples of the preferred nonionic surface active agents may include polyoxyethylene sorbitan fatty acid esters such as polysorbate 20, 60 and 80; polyoxyethylene castor oil derivatives such as polyoxyethylene castor oil 35, polyoxyethylene hydrogenated castor oil.40 and polyoxyethylene hydrogenated castor oil 60; polyoxyethylene alkylethers, polyoxyethylene polyoxypropyleneglycols; and polyoxyl stearates.

The pharmaceutical composition of the present invention may further comprise an additive, for example, anti oxidant such as ethylenediaminetetraacetic acid (EDTA); buffering agent such as boric acid, borax and citric acid; preserving agent such as benzalkonium chloride, benzethonium chloride and chlorhexidine gluconate. Benzalkonium chloride is preferable. The amount of the preserving agent in the pharmaceutical composition of the present invention may be about 0.001-0.05w/v%, preferably, about 0.002-0.02w/v% based on the total volume of the composition.

The pharmaceutical composition of the present invention may comprise sole active ingredient or a combination of two or more active ingredients. When two or more active ingredients are used together, the amount of each active ingredient may be increased or decreased in view of its therapeutic effect and safety.

The composition of the present invention may further comprise the other active ingredient in so far as it does not act adverse to the purpose of the present invention.

According to the present invention, by combining a PG compound with a sugar alcohol and a polyol, a highly stable pharmaceutical composition could be provided. The composition can stably be stored even in a polyethylene container while the conventional composition comprising a PG compound and a sugar alcohol could not be stored in a polyethylene container because of the poor stability. In addition, the pharmaceutical composition of the present invention has good preserving property even if the composition comprises lower amount of the preserving agent.

The present invention will be explained in more detail by means of the following examples, which are illustrated by way of example only and never intended to limit the scope of the present invention.

### EXAMPLE 1

Test solution 1 was obtained by dissolving the ingredients in an amount shown below (w/v%) in purified water and sterilized by filter sterilization.
Compound A: 13,14-dihydro-15-keto-20-ethyl-PGF2α isopropyl ester was used.

| | |
|---|---|
| 0.12% | Compound A |
| 1.0% | polysorbate 80 |
| 1.0% | mannitol |
| 1.9% | glycerine |
| 0.05% | edetate disodium |
| 0.003% | benzalkonium chloride |

Thus obtained test solution 1 was filled in a sterilized low density polyethylene (LDPE) container under sterile condition. The container was kept at 25°C for 12 months or at 40°C for 6 months. After that, the concentration of the compound A in the test solution was determined with a liquid chromatograph. Results are shown in Tables 1 and 2.

**Table 1**

| Stability of compound A: stored at 40°C/25%RH or lower for 6 months | | |
|---|---|---|
| | conc. (% of day 0) | |
| | day 0 | 6 months |
| test solution 1 | 100 | 99.3 |

**Table 2 Stability of compound A: stored at 25°C/40%RH for 6/12 months**

| | conc. (% of day 0) | | |
|---|---|---|---|
| | day 0 | 6 months | 12 months |
| test solution 1 | 100 | 99.0 | 99.3 |

As shown above, compound A in the test solution 1 is stable when the solution is stored in the low density polyethylene container and therefore, the composition very stable and can be stored for long term at room temperature.

### COMPARATIVE EXAMPLE 1

Test solution 2 was prepared by the same manner as Example 1 using the ingredients shown below:

| | |
|---|---|
| 0.12% | compound A |
| 1.0% | polysorbate 80 |
| 4.86% | mannitol |
| 0.1% | edetate disodium |
| 0.01% | benzalkonium chloride |

### COMPARATIVE EXAMPLE 2

Test solution 3 was prepared by the same manner as Example 1 using the ingredients shown below:

| | |
|---|---|
| 0.12% | compound A |
| 1.0% | polysorbate 80 |
| 2.43% | mannitol |
| 0.43% | sodium chloride |
| 0.1% | edetate disodium |
| 0.01% | benzalkonium chloride |

The obtained test solutions 2 and 3 were filled in a sterilized LDPE container and stored at 55°C for 1 month. After that, the concentration of the compound A in the test solution was determined by a liquid chromatograph. Results are shown in Table 3.

**Table 3**

| Stability of compound A: stored at 55°C | | |
|---|---|---|
| | conc. (% of day 0) | |
| | day 0 | 1 month |
| test solution 2 | 100 | 91.6 |
| test solution 3 | 100 | 90.8 |

According to the result of comparative examples 1 and 2, the test solutions 2 and 3 that comprise no glycerine could not achieve the satisfying stability.

### EXAMPLE 2

Test solution 4 was prepared by the same manner as Example 1 using the ingredients shown below:

| | |
|---|---|
| 0.12% | Compound A |
| 1.0% | polysorbate 80 |
| 2.0% | mannitol |
| 1.49% | glycerine |
| 0.05% | edetate disodium |
| 0.005% | benzalkonium chloride |

### COMPARATIVE EXAMPLE 3

Test solution 5 was prepared by the same manner as Example 1 using the ingredients shown below:

| | |
|---|---|
| 0.12% | Compound A |
| 1.0% | polysorbate 80 |
| 2.0% | mannitol |
| 0.52% | sodium chloride |
| 0.05% | sodium edetate |
| 0.01% | benzalkonium chloride |

Thus obtained test solutions 4 and 5 was filled in a sterilized container under sterile condition and *Pseudomonas aeruginosa* was inoculated to the container and mixed uniformly. The container was kept at 20-25°C and the viable cell count was determined at 14 and 28days after the inoculation. The viable cell count determination was conducted by means of the agar plate diffusion test. The log reduction value of the cell number from the inoculated number of the cells over time were calculated. Results are shown in Table 4.

**Table 4**

| log reduction of cell number | | | |
|---|---|---|---|
| | BAC (%) | log reduction of cell number | |
| | | 14 days | 28 days |
| test solution 4 | 0.005 | ND | ND |
| test solution 5 | 0.01 | -1.2 log | -0.51 log |

According to the result, test solution 4 comprising mannitol and glycerine can keep good antibacterial activity though the lower concentration of benzalkonium chloride.

## Claims

1. A pharmaceutical composition comprising:
(a) a prostaglandin compound of formula (II): 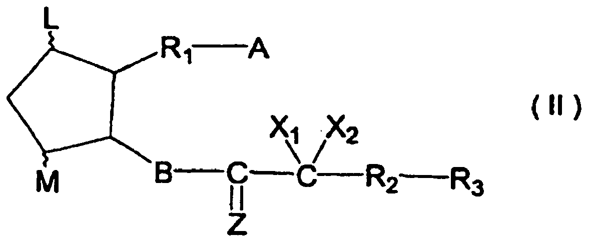 wherein L and M are hydrogen, hydroxy, halogen, lower alkyl, hydroxy(lower)alkyl or oxo, wherein at least one of L and M is a group other than hydrogen, and the five-membered ring may have at least one double bond;
A is -CH₃, -CH₂OH, -COCH₂OH, -COOH or a functional derivative thereof;
B is single bond, -CH₂-CH₂-, -CH=CH-, -C≡C-, -CH₂-CH₂-CH₂-, -CH=CH-CH₂-, -CH₂-CH=CH-, -C=C-CH₂- or -CH₂-C≡C-;
Z is 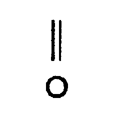 X₁ and X₂ are hydrogen, lower alkyl, or halogen;
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one of carbon atom in the aliphatic hydrocarbon is optionally substituted by oxygen, nitrogen or sulfur;
R₂ is single bond or lower alkylene; and
R₃ is lower alkyl, lower alkoxy, cyclo(lower)alkyl, cyclo(lower)alkyloxy, aryl, aryloxy, heterocyclic group or heterocyclic-oxy group,
(b) a sugar alcohol, which is selected from the group consisting of mannitol, sorbitol, maltitol, sugar alcohol solution derived from corn starch and hydrogenated maltose starch syrup.
(c) a polyol, which is selected from the group consisting of glycerine, polyethyleneglycol and propyleneglycol, and
(d) a pharmaceutically acceptable carrier
wherein the composition is stored in a polyethylene container.

2. The pharmaceutical composition of Claim 1, wherein the prostaglandin compound is isopropyl unoprostone.

3. The pharmaceutical composition of Claim 1, further comprising a non-ionic surface active agent.

4. The pharmaceutical composition of Claim 1, for use in the treatment of glaucoma and/or ocular hypertension or retinitis pigmentosa.

5. The pharmaceutical composition of Claim 1, wherein the sugar alcohol is mannitol.

6. The pharmaceutical composition of Claim 1, wherein polyol is glycerine.

7. The pharmaceutical composition of Claim 1, which is in a dosage form suitable for ocular topical administration.

8. The pharmaceutical composition of Claim 1, which is formulated as eye drops.

9. The pharmaceutical composition of Claim 1, wherein the polyethylene container is made of low density polyethylene.

10. The pharmaceutical composition of Claim 1, wherein the pharmaceutically acceptable carrier is water.

## Patentansprüche

1. Pharmazeutische Zusammensetzung, umfassend:
(a) eine Prostaglandin-Verbindung der Formel (II): 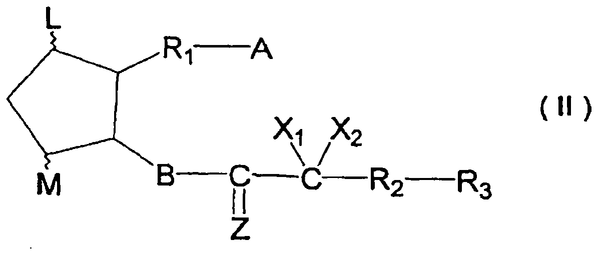 worin L und M Wasserstoff, Hydroxy, Halogen, Niederalkyl, Hydroxy(nieder)alkyl oder Oxo sind, wobei wenigstens eins von L und M eine andere Gruppe als Wasserstoff ist und der fünfgliedrige Ring wenigstens eine Doppelbindung haben kann;
A -CH₃, -CH₂OH, -COCH₂OH, -COOH oder ein funktionelles Derivat davon ist;
B eine Einfachbindung, -CH₂-CH₂-, -CH=CH-, -C≡C-, -CH₂-CH₂-CH₂-, -CH=CH-CH₂-, -CH₂-CH=CH-, -C≡C-CH₂- oder -CH₂-C≡C- ist;
Z 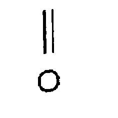 ist;
X₁ und X₂ Wasserstoff, Niederalkyl oder Halogen sind;
R₁ ein gesättigter oder ungesättigter divalenter niederer oder mittlerer aliphatischer Kohlenwasserstoffrest ist, der unsubstituiert oder mit einer Halogen-, Niederalkyl-, Hydroxy-, Oxo-, Aryl- oder heterocyclischen Gruppe substituiert ist, und wobei wenigstens ein Kohlenstoffatom in dem aliphatischen Kohlenwasserstoff gegebenenfalls durch Sauerstoff, Stickstoff oder Schwefel ersetzt ist;
R₂ eine Einfachbindung oder Niederalkylen ist und
R₃ eine Niederalkyl-, Niederalkoxy-, Cyclo(nieder)alkyl-, Cyclo(nieder)alkyloxy-, Aryl-, Aryloxy-, heterocyclische Gruppe oder heterocyclische Oxygruppe ist,
(b) einen Zuckeralkohol, der aus der Gruppe, bestehend aus Mannit, Sorbit, Maltit, Zuckeralkohollösung, die von Maisstärke und hydriertem Maltosestärkeserum abgeleitet ist, ausgewählt ist;
(c) ein Polyol, das aus der Gruppe, bestehend aus Glycerin, Polyethylenglycol und Propylenglycol, ausgewählt ist, und
(d) einen pharmazeutisch verträglichen Träger,
wobei die Zusammensetzung in einem Polyethylenbehälter gelagert wird.

2. Pharmazeutische Zusammensetzung gemäß Anspruch 1, wobei die Prostaglandin-Verbindung Isopropylunoproston ist.

3. Pharmazeutische Zusammensetzung gemäß Anspruch 1, die außerdem ein nichtionisches oberflächenaktives Mittel umfasst.

4. Pharmazeutische Zusammensetzung gemäß Anspruch 1 zur Verwendung bei der Behandlung von Glaukom und/oder Augenhochdruck oder Retinitis pigmentosa.

5. Pharmazeutische Zusammensetzung gemäß Anspruch 1, wobei der Zuckeralkohol Mannit ist.

6. Pharmazeutische Zusammensetzung gemäß Anspruch 1, wobei Polyol Glycerin ist.

7. Pharmazeutische Zusammensetzung gemäß Anspruch 1, welche in einer Dosierungsform ist, die zur okularen topischen Verabreichung geeignet ist.

8. Pharmazeutische Zusammensetzung gemäß Anspruch 1, die als Augentropfen formuliert ist.

9. Pharmazeutische Zusammensetzung gemäß Anspruch 1, wobei der Polyethylenbehälter aus Polyethylen niedriger Dichte hergestellt ist.

10. Pharmazeutische Zusammensetzung gemäß Anspruch 1, wobei der pharmazeutisch verträgliche Träger Wasser ist.

## Revendications

1. Composition pharmaceutique comprenant :
(a) un composé prostaglandine de formule (II) : 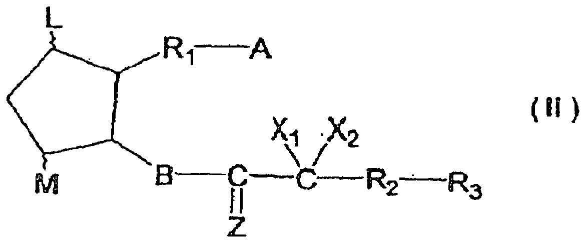 dans laquelle L et M sont un groupe hydrogène, hydroxy, halogène, alkyle inférieur, hydroxy(alkyle inférieur) ou oxo, dans laquelle au moins l'un de L et M est un groupe autre qu'un hydrogène, et le cycle à cinq chaînons peut avoir au moins une double liaison,
A est -CH₃, -CH₂OH, -COCH₂OH, -COOH ou un dérivé fonctionnel de ceux-ci ;
B est une liaison simple, -CH₂-CH₂-, -CH=CH-, - C≡C-, -CH₂-CH₂-CH₂-, -CH=CH-CH₂-, -CH₂-CH=CH-, -C≡C-CH₂-ou -CH₂-C≡C-;
Z est 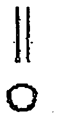 X₁ et X₂ sont un groupe hydrogène, alkyle inférieur, ou halogène ;
R₁ est un résidu hydrocarbure aliphatique inférieur ou moyen, bivalent, saturé ou insaturé, qui est substitué ou non substitué par un atome d'halogène, un groupe alkyle inférieur, hydroxy, oxo, aryle or hétérocyclique, et au moins un atome de carbone dans le groupe hydrocarbure aliphatique est facultativement substitué par un atome d'oxygène, d'azote ou de soufre ;
R₂ est une liaison simple ou un groupe alkylène inférieur ; et
R₃ est un groupe alkyle inférieur, alcoxy inférieur, cyclo-(alkyle inférieur), cyclo-(alkyloxy inférieur), aryle, aryloxy, hétérocyclique ou hétérocyclique-oxy,
(b) un sucre glucidique qui est choisi dans le groupe constitué du mannitol, du sorbitol, du maltitol, d'une solution de sucre glucidique dérivée d'amidon de maïs et d'un sirop d'amidon de maltose hydrogéné,
(c) un polyol, qui est choisi dans le groupe constitué de la glycérine, du polyéthylèneglycol et du propylèneglycol, et
(d) un vecteur pharmaceutiquement acceptable,
dans laquelle la composition est conservée dans un récipient en polyéthylène.

2. Composition pharmaceutique selon la revendication 1, dans laquelle le composé prostaglandine est l'isopropyl unoprostone.

3. Composition pharmaceutique selon la revendication 1, comprenant en outre un agent tensioactif non ionique.

4. Composition pharmaceutique selon la revendication 1, destinée à être utilisée dans le traitement du glaucome et/ou de l'hypertension oculaire ou de la rétinite pigmentaire.

5. Composition pharmaceutique selon la revendication 1, dans laquelle l'alcool glucidique est le mannitol.

6. Composition pharmaceutique selon la revendication 1, dans laquelle le polyol est la glycérine.

7. Composition pharmaceutique selon la revendication 1, qui se trouve sous une forme galénique adaptée à une administration oculaire topique.

8. Composition pharmaceutique selon la revendication 1, qui est formulée sous la forme de gouttes oculaires.

9. Composition pharmaceutique selon la revendication 1, dans laquelle le récipient en polyéthylène est constitué de polyéthylène basse densité.

10. Composition pharmaceutique selon la revendication 1, dans laquelle le support pharmaceutiquement acceptable est l'eau.
